# EUROPEAN PATENT SPECIFICATION

(11) **EP 0 372 527 B1**
(45) Date of publication and mention of the grant of the patent: **12.05.1993**
(21) Application number: 89122486.7
(22) Date of filing: 06.12.1989
(51) Int. Cl.: A61K 31/195, A61K 47/14, A61K 47/24, A61K 47/32, A61K 47/00

(54) **Topical compositions containing diclofenac**
Topisch anzuwendende Diclofenac enthaltende Arzneizubereitungen
Compositions à usage topique contenant le diclofénac

(30) Priority: 09.12.1988 IT 2290288
(43) Date of publication of application: 13.06.1990
(73) Proprietor: ALTERGON S.A., CH-6900 Lugano (CH)
(72) Inventor: Donati-Pedemonti, Elisabetta, I-22100 Como (IT); Lualdi, Paolo, I-22070 Grandate(Como) (IT)
(74) Representative: Gervasi, Gemma, Dr.

(56) References cited:
- EP-A- 0 271 709
- CHEMICAL ABSTRACTS, vol. 105, no. 12, 22nd September 1986, page 336, abstract no. 102589c, Columbus, Ohio, US

## Description

Those substances which possess maximum cutaneous absorption levels are known to present amphipathic characteristics of between 4 and 15 expressed as the HLB (hydrophil-lipophil balance) value (Remington's Pharmaceutical Sciences, Easton Penna, 1975, USA) and a very low degree of ionization.

EP-A-271.709 discloses diclofenac salt of hydroxyethylpyrrolidine (DIEP) which is soluble in water.

Diclofenac hydroxyethylpyrrolidine (DIEP) is soluble in water to concentrations exceeding 50% (w/v) in which it ionizes practically totally, but is only slightly soluble in polar organic solvents, and is practically insoluble in apolar organic solvents.

These physico-chemical characteristics mean that a product dissolved in a water phase cannot be satisfactorily absorbed by the cutis, as demonstrated by the insufficient antiinflammatory activity obtained when DIEP is applied in the form of medicated gels based on cellulose or acrylic derivatives, such as those of the following compositions.

| Composition I: | |
|---|---|
| DIEP | 1.32% |
| Neutralized polymerized acrylic acid | 2.00% |
| Isopropyl alcohol | 10.00% |
| Demineralized water to make up to | 100.00% |

| Composition II: | |
|---|---|
| DIEP | 1.32% |
| Glycerin | 25.00% |
| Hydroxyethylcellulose | 2.00% |
| Parabens | 0.80% |
| Demineralized water to make up to | 100.00% |

In fact EP-A-271.709 describes pharmaceutical formulations of DIEP for oral use only.

We have now discovered new pharmaceutical compositions for topical use able to act as improved vehicles for diclofenac hydroxyethylpyrrolidine (DIEP). Said compositions comprise:
diclofenac hydroxyethylpyrrolidine (DIEP), lipid substances of amphipathic character with high cutaneous absorption, suitable surfactants, co-solvents and additives of common pharmaceutical use suitable for topical application, incorporated in a viscous hydrophilic support (gel).

Said compositions are prepared by dissolving the DIEP in the lipid substances in the presence of surfactants, co-solvents and additives, and emulsifying the obtained mixture together with the viscous hydrophilic support.

The compositions obtained have physico-chemical characteristics which indicate their suitability for cutaneous absorption and are easy to apply locally.

The characteristics and advantages of the pharmaceutical compositions for topical use according to the invention will be more apparent from the following detailed description.

Said compositions comprise diclofenac hydroxyethylpyrrolidine (DIEP) as active principle and, as vehicles, lipid substances of amphipathic character, surfactants, co-solvents and additives of common pharmaceutical use, and are incorporated in a viscous hydrophilic support (gel).

Of the possible lipid substances which can be used, those pertaining to the following four groups are preferred:
1) Cetyl and stearyl esters of ethylhexanoic acid which have been made hydrophilic by surfactants, and have an HLB of between 10 and 12;
2) C₈-C₁₈ mono and/or di and/or triglycerides with different polyoxyethylenated/glycolyzed aliphatic chains, and having an HLB of between 4 and 14;
3) Phospholipids of vegetable origin having an HLB of between 12 and 14;
4) Lanolin esters having an HLB of 9.

The dissolving of the DIEP in said lipid compounds is aided by adding non-ionic surfactants having an HLB of between 10 and 13, such as polyethyleneglycol stearates, cetomacrogols etc.

The co-solvents used can be glycols, ethyldiglycol or low molecular-weight polyethyleneglycols, which also perform a wetting action in the complete formulation.

The lipid solutions obtained are emulsified in a transparent hydrophilic gel consisting of suitably neutralized polymerized acrylic acid. A gel is obtained having a milky or transparent appearance, and of suitable viscosity and consistency for application to the skin.

The composition is completed by the addition of additives of common pharmaceutical use such as antimicrobials, for example nipaginics, isopropyl alcohol or perfumes.

In the compositions of the present invention the various components are contained in the following weight proportions: DIEP between 0.5 and 2%, lipids between 1 and 5%, surfactants between 1 and 10%, co-solvents between 3 and 12%, acrylic acid between 0.5 and 3%, and other commonly used additives between 7 and 15%, the difference to 100% being demineralized water.

When used topically, the compositions prepared in this manner are easily absorbed to carry the DIEP into the dermis where it efficiently performs its pharmacological action.

The following examples of compositions according to the present invention are given for illustrative purposes.

| EXAMPLE 1 | |
|---|---|
| | % by weight |
| DIEP | 0.5- 2 |
| acetylstearyl-2-ethylhexanoate | 1 - 4 |
| polyethyleneglycol 400 stearate | 1 - 2 |
| polyethyleneglycol 300 or propyleneglycol | 5 - 10 |
| polymerized acrylic acid | 0.5- 3 |
| triethanolamine | 1 - 4 |
| isopropanol | 6 - 10 |
| perfume | 0.1- 0.2 |
| demineralized water to make up to | 100 |

| EXAMPLE 2 | |
|---|---|
| | % by weight |
| DIEP | 0.5- 2 |
| cetylstearyl-2-ethylhexanoate | 1 - 2 |
| cetomacrogol stearyl alcohol | 1 - 2 |
| polyethyleneglycol 300 or propyleneglycol | 5 - 10 |
| polymerized acrylic acid | 0.5- 3 |
| triethanolamine | 1 - 4 |
| isopropanol | 6 - 10 |
| perfume | 0.1- 0.2 |
| demineralized water to make up to | 100 |

| EXAMPLE 3 | |
|---|---|
| | % by weight |
| DIEP | 0.5- 2 |
| polyoxyethylenated C₁₂-C₁₈ glycerides | 1 - 4 |
| ethyldiglycol | 3 - 8 |
| polyethyleneglycol 400 stearate | 1 - 2 |
| polymerized acrylic acid | 0.5- 3 |
| triethanolamine | 1 - 4 |
| isopropanol | 6 - 10 |
| perfume | 0.1- 0.2 |
| demineralized water to make up to | 100 |

| EXAMPLE 4 | |
|---|---|
| | % by weight |
| DIEP | 0.5- 2 |
| polyoxyethylenated ricinoleic triglyceride | 0.5- 5 |
| polyethyleneglycol 400 stearate | 1 - 2 |
| propyleneglycol | 5 - 10 |
| polymerized acrylic acid | 0.5- 3 |
| triethanolamine | 1 - 4 |
| isopropanol | 6 - 10 |
| perfume | 0.1- 0.2 |
| demineralized water to make up to | 100 |

| EXAMPLE 5 | |
|---|---|
| | % by weight |
| DIEP | 0.5- 2 |
| soya lecithin | 2 - 5 |
| polyethyleneglycol 400 stearate | 1 - 2 |
| glycerin | 5 - 10 |
| polymerized acrylic acid | 0.5- 3 |
| triethanolamine | 1 - 4 |
| isopropanol | 6 - 10 |
| perfume | 0.1- 0.2 |
| demineralized water to make up to | 100 |

| EXAMPLE 6 | |
|---|---|
| | % by weight |
| DIEP | 0.5- 2 |
| isopropyl lanolate | 1 - 4 |
| glyceryl monostearate or polyethyleneglycol 400 stearate | 1 - 4 |
| propyleneglycol | 6 - 12 |
| polymerized acrylic acid | 0.5- 3 |
| triethanolamine | 1 - 4 |
| isopropanol | 6 - 10 |
| perfume | 0.1- 0.2 |
| dimineralized water to make up to | 100 |

By way of example, the antiinflammatory activity results obtained experimentally in the rat by applying 1 gram of some of the aforesaid preparations indicated with 1-4-5 are shown hereinafter, compared with those of a hydrophilic gel containing the same concentration of DIEP (Composition I of the known art).

| Preparation | DIEP concentration | Quantity applied to rat | No. of rats | Analgesic/peripheral anti-inflammatory activity (Randall-Selitto test) |
|---|---|---|---|---|
| 1 | 1.32% | 1 g | 5 | 65% |
| 4 | 1.32% | 1 g | 5 | 72% |
| 5 | 1.32% | 1 g | 5 | 68% |
| Hydrophilic gel (Composition I) | 1.32% | 1 g | 5 | 22% |

These results clearly indicate the considerable increase in absorption of the active principle obtained with the compositions according to the present invention.

## Claims (Claims for the following Contracting State(s): AT, BE, CH, DE, FR, GB, IT, LI, LU, NL, SE)

1. Pharmaceutical compositions for topical use able to act as vehicles for diclofenac hydroxyethylpyrrolidine (DIEP) characterized in that they comprise:
a) diclofenac hydroxyethylpyrrolidine (DIEP) as active principle;
b) lipid substances of amphipathic character with high cutaneous absorption, selected from the classis of
- cetyl and stearyl esters of ethylhexanoic acid made hydrophilic by surfactants and havig a HLB between 10 and 12;
- C₈ ÷ C₁₈ mono- and/or di- and/or tri-glycerides with different poly oxyethylenated/glycolyzed aliphatic chains, with an HLB of between 4 and 14;
- phospholipids of vegetable origin with an HLB between 12 and 14;
- lanolin esters with an HLB of 9;
c) a surfactant of non-ionic type with an HLB of between 10 and 13
d) a viscous hydrophilic support consisting of neutralized polymerized acrylic acid
e) co-solvents selected from: glycols, ethyldiglycol and low molecular weight polyethyleneglycols.

2. Compositions as claimed in claim 1, characterised in that said surfactants are polyethyleneglycol stearates and cetomacrogols.

3. Compositions as claimed in claim 1, characterised by comprising the various components in the following weight proportions: DIEP between 0.5 and 2%, lipids between 1 and 5%, surfactants between 1 and 10%, co-solvents between 3 and 12%, acrylic acid between 0,5 and 3% and other commonly used additives between 7 and 15%, the difference to 100% being demineralized water.

4. A method for preparing the compositions claimed in claim 1, characterised in that the DIEP is dissolved in the lipid substances in the presence of the surfactants, co-solvents and additives, the mixture obtained then being emulsified with the viscous hydrophilic support.

## Claims (Claims for the following Contracting State(s): ES, GR)

1. Process for preparing pharmaceutical compositions for topical use, containing as active principle diclofenac hydroxyethylpyrrolidine (DIEP), which comprises the following steps:
a) dissolving DIEP in a lipid substance of amphipathic character having high cutaneous absorption, selected from the classis of:
- cetyl and stearyl esters of ethylhexanoic acid made hydrophilic by surfactants and having a HLB between 10 and 12;
- C₈ +C₁₈ mono- and/or di- and/or tri-glycerides with different poly oxyethylenated/glycolyzed aliphatic chains, with an HLB of between 4 and 14;
- phospholipids of vegetable origin with an HLB between 12 and 14;
- lanolin esters with a HLB of 9;
in combination with a co-solvent selected from:
- glycols, ethyldiglycol and low molecular weight polyethylenglycols
and with a surfactant of non-ionic type having an HLB between 10 and 13;
b) emulsifying the lipid solution of DIEP with a hydrophilic gel consisting of neutralized polymerized acrylic acid.

2. Process according to claim 1 wherein the various components are used in the following weight proportions:
DIEP between 0.5 and 2%, lipids between 1 and 5%, surfactants beween 1 and 10%, co-solvents between 3 and 12%, acrylic acid between 0.5 and 3% and furthermore commonly used additives, the difference to 100% being demineralized water, and commonly used additives.

3. Process according to claim 1 wherein the surfactant of non-ionic type are selected from polyethylenglycol stearates and cetomacrogols.

## Patentansprüche (Patentansprüche für folgende(n) Vertragsstaat(en): AT, BE, CH, DE, FR, GB, IT, LI, LU, NL, SE)

1. Pharmazeutische Zusammensetzungen für die topische Verwendung, die als Vehicula für Diclofenac-Hydroxyethylpyrrolidin (DIEP) wirken können, dadurch gekennzeichnet, daß sie enthalten:
a) Diclofenac-Hydroxyethylpyrrolidin (DIEP) als aktives Prinzip (Wirkstoff);
b) Lipid-Substanzen mit amphipathischem Charakter mit einer hohen Hautabsorption, ausgewählt aus der Klasse, die besteht aus
- Cetyl- und Stearylestern der Ethylhexansäure, die durch oberflächenaktive Agentien hydrophil gemacht worden sind und einen HLB-Wert zwischen 10 und 12 aufweisen;
- C₈-C₁₈-Mono- und/oder -Di- und/oder -Triglyceriden mit unterschiedlichen polyoxyethylenierten/glycolysierten aliphatischen Ketten mit einem HLB-Wert zwischen 4 und 14;
- Phospholipiden Pflanzlichen Ursprungs mit einem HLB-Wert zwischen 12 und 14;
- Lanolinestern mit einem HLB-Wert von 9;
c) ein oberflächenaktives Agens vom nicht-ionischen Typ mit einem HLB-Wert zwichen 10 und 13,
d) einen viskosen hydrophilen Träger, der aus neutralisieter polymerisierter Acrylsäure besteht,
e) Colösungsmittel, ausgewählt aus Glycolen, Ethyldiglycol und Polyethylenglycolen mit niedrigem Molekulargewicht.

2. Zusammensetzungen nach Anspruch 1, dadurch gekennzeichnet, daß es sich bei den oberflächenaktiven Agentien um Polyethylenglycolstearate und Ketomacrogole handelt.

3. Zusammensetzungen nach Anspruch 1, dadurch gekennzeichnet, daß sie die verschiedenen Komponenten in den folgenden Gewichtsmengenanteilen enthalten:
DIEP zwischen 0,5 und 2 %, Lipide zwischen 1 und 5 %, oberflächenaktive Agentien zwischen 1 und 10 %, Colösungsmittel zwischen 3 und 12 %, Acrylsäure zwischen 0,5 und 3 % und andere üblicherweise verwendete Additive zwischen 7 und 15 %, wobei die Differenz zu 100 % entmineralisiertes Wasser ist.

4. Verfahren zur Herstellung der Zusammensetzungen nach Anspruch 1, dadurch gekennzeichnet, daß das DIEP in den Lipid-Substanzen gelöst wird in Gegenwart der oberflächenaktiven Agentien, der Colösungsmittel und der Additive, und daß die dabei erhaltene Mischung dann mit dem viskosen hydrophilen Träger emulgiert wird.

## Patentansprüche (Patentansprüche für folgende(n) Vertragsstaat(en): ES, GR)

1. Verfahren zur Herstellung von pharmazeutischen Zusammensetzungen für die topische Verwendung, die als aktives Prinzip (Wirkstoff) Diclofenac-Hydroxyethylpyrrolidin (DIEP) enthalten, das die folgenden Stufen umfaßt:
a) Auflösen von DIEP in einer Lipid-Substanz mit amphipathischem Charakter mit einer hohen Hautabsorption, ausgewählt aus der Klasse, die besteht aus
- Cetyl- und Stearylestern der Ethylhexansäure, die durch oberflächenaktive Agentien hydrophil gemacht worden sind und einen HLB-Wert zwischen 10 und 12 aufweisen;
- C₈-C₁₈-Mono- und/oder -Di- und/oder -Triglyceriden mit unterschiedlichen polyoxyethylenierten/glycolysierten aliphatischen Ketten mit einem HLB-Wert zwischen 4 und 14;
- Phospholipiden pflanzlichen Ursprungs mit einem HLB-Wert zwischen 12 und 14;
- Lanolinestern mit einem HLB-Wert von 9;
in Kombination mit einem Colösungsmittel, ausgewählt aus Glycolen, Ethyldiglycol und Polyethylenglycolen mit niedrigem Molekulargewicht und
mit einem oberflächenaktiven Agens vom nicht-ionischen Typ mit einem HLB-Wert zwischen 10 und 13;
b) Emulgieren der Lipid-Lösung von DIEP mit einem hydrophilen Gel, das aus neutralisierter polymerisierter Acrylsäure besteht.

2. Verfahren nach Anspruch 1, worin die verschiedenen Komponenten in den folgenden Gewichtsmengenanteilen verwendet werden:
DIEP zwischen 0,5 und 2 %, Lipide zwischen 1 und 5 %, oberflächenaktive Agentien zwischen 1 und 10 %, Colösungsmittel zwischen 3 und 12 %, Acrylsäure zwischen 0,5 und 3 % und außerdem üblicherweise verwendete Additive, wobei die Differenz zu 100 % entmineralisiertes Wasser ist, und üblicherweise verwendete Additive.

3. Verfahren nach Anspruch 1, worin die oberflächenaktiven Agentien vom nicht-ionischen Typ ausgewählt werden aus Polyethylenglycolstearaten und Ketomacrogolen.

## Revendications (Revendications pour l'(les) Etat(s) contractant(s) suivant(s): AT, BE, CH, DE, FR, GB, IT, LI, LU, NL, SE)

1. Compositions pharmaceutiques à usage topique, susceptibles de servir de véhicules pour la diclofénac-hydroxyéthylpyrrolidine (DIEP), caractérisées en ce qu'elles comprennent:
a) de la diclofénac-hydroxyéthylpyrrolidine (DIEP) en tant que principe actif;
b) des substances lipidiques de caractère amphipathique à forte absorption cutanée, choisies dans la classe
- des esters cétyliques et stéaryliques d'acide éthylhexanoïque, rendus hydrophiles par des surfactants et ayant un indice d'équilibre hydrophile-lipophile (HLB) entre 10 et 12;
- des mono- et/ou triglycérides en C₈-C₁₈ contenant des chaînes aliphatiques polyoxyéthylénées/glycolysées différentes et ayant un HLB entre 4 et 14;
- des phospholipides d'origine végétale, ayant un HLB entre 12 et 14;
- des esters de lanoline ayant un HLB de 9;
c) un surfactant de type non ionique, ayant un HLB entre 10 et 13;
d) un support hydrophile visqueux, constitué par un acide acrylique polymérisé neutralisé;
e) des co-solvants choisis parmi les glycols, l'éthyldiglycol et les polyéthylèneglycols de bas poids moléculaire.

2. Compositions selon la revendication 1, caractérisées en ce que lesdits surfactants sont des stéarates de polyéthylèneglycol et des cétomacrogols.

3. Compositions selon la revendication 1, caractérisées en ce qu'elles contiennent les différents constituants dans les proportions en poids suivantes: DIEP, entre 0,5 et 2%; lipides, entre 1 et 5%; surfactants, entre 1 et 10%; co-solvants, entre 3 et 12%; acide acrylique, entre 0,5 et 3%; et autres additifs utilisés couramment, entre 7 et 15%; la différence à 100% étant faite d'eau déminéralisée.

4. Procédé de préparation des compositions selon la revendication 1, caractérisé en ce que la DIEP est dissoute dans les substances lipidiques en présence des surfactants, des co-solvants et des additifs, le mélange obtenu étant ensuite émulsionné avec le support hydrophile visqueux.

## Revendications (Revendications pour l'(les) Etat(s) contractant(s) suivant(s): ES, GR)

1. Procédé de préparation de compositions pharmaceutiques à usage topique contenant, en tant que principe actif, de la diclofénac-hydroxyéthylpyrrolidine (DIEP), comprenant les étapes consistant
a) à dissoudre de la DIEP dans une substance lipidique de caractère amphipathique à forte absorption cutanée, choisie dans la classe
- des esters cétyliques et stéaryliques d'acide éthylhexanoïque, rendus hydrophiles par des surfactants et ayant un indice d'équilibre hydrophile-lipophile (HLB) entre 10 et 12;
- des mono- et/ou triglycérides en C₈-C₁₈ contenant des chaînes aliphatiques polyoxyéthylénées/glycolysées différentes et ayant un HLB entre 4 et 14;
- des phospholipides d'origine végétale, ayant un HLB entre 12 et 14;
- des esters de lanoline ayant un HLB de 9;
en combinaison avec un co-solvant choisi parmi
- les glycols, l'éthyldiglycol et les polyéthylèneglycols de bas poids moléculaire
et avec un surfactant de type non ionique ayant un HLB entre 10 et 13; et
b) à émulsionner la solution lipidique de DIEP avec un gel hydrophile constitué par un acide acrylique polymérisé neutralisé.

2. Procédé selon la revendication 1, dans lequel les différents constituants sont utilisés dans les proportions en poids suivantes: DIEP, entre 0,5 et 2%; lipides, entre 1 et 5%; surfactants, entre 1 et 10%; co-solvants, entre 3 et 12%; acide acrylique, entre 0,5 et 3%; et, en outre, des additifs utilisés couramment; la différence à 100% étant faite d'eau déminéralisée et des additifs utilisés couramment.

3. Procédé selon la revendication 1, dans lequel le surfactant de type non ionique est choisi parmi les stéarates de polyéthylèneglycol et les cétomacrogols.
